(19) Europäisches Patentamt
European Patent Office
Office européen des brevets

(11) **EP 3 406 192 A1**

(12) **EUROPEAN PATENT APPLICATION**
published in accordance with Art. 153(4) EPC

(43) Date of publication:
**28.11.2018 Bulletin 2018/48**

(51) Int Cl.:
***A61B 5/05*** *(2006.01)*

(21) Application number: **15908303.9**

(86) International application number:
**PCT/JP2015/081812**

(22) Date of filing: **12.11.2015**

(87) International publication number:
**WO 2017/081783 (18.05.2017 Gazette 2017/20)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Matrix Cell Research Institute Inc.
Ushiku-shi, Ibaraki 300-1232 (JP)**

(72) Inventors:
• **KUSAKABE Moriaki
Ushiku-shi
Ibaraki 300-1232 (JP)**

• **SEKINO Masaki
Tokyo 151-0064 (JP)**
• **OOKUBO Tetsu
Funabashi-shi
Chiba 273-0031 (JP)**
• **SAITO Itsuro
Tokyo 120-0022 (JP)**
• **MAEDA SHINSAKU
Nasushiobara-shi
Tochigi 325-0033 (JP)**

(74) Representative: **Isarpatent
Patent- und Rechtsanwälte Behnisch Barth
Charles
Hassa Peckmann & Partner mbB
Friedrichstrasse 31
80801 München (DE)**

(54) **MAGNETIC FLUID DETECTION DEVICE**

(57) It is an object of the invention to facilitate confirmation of detection results while allowing magnetic fluid detection operation to be carried out without interference from a cable.

The solution is a magnetic fluid detecting device 1 that detects magnetic fluid that has been injected into a living body, the device comprising a detector 2 that detects magnetic fluid in the body when it is in contact with or adjacent to the body, an output device 3 that outputs the detection results in a prescribed output form, a controller 4 that controls the output device 3 based on the detected value inputted from the detector 2, and a power supply module 5 that supplies power to the detector 2, output device 3 and controller 4, using a battery B as the power source, the detector 2, output device 3, controller 4 and power supply module 5 being integrated so as to allow holding with one hand.

FIG.2

**Description**

Technical Field

The present invention relates to a magnetic fluid detecting device to be used for detection of magnetic fluids injected into a living body.

Background Art

Cancer cells in solid malignant tumors are currently known to metastasize throughout the body via lymphatic vessels, and cancer cells that have infiltrated lymphatic vessels become captured in the intervening lymph nodes. When a tumor has been discovered in the clinic, a "sentinel lymph node" is identified as a lymph node that is located downstream in the lymph flow from the lesion site and has lymph flowing in from the lesion site, and during surgical operation, the identified sentinel lymph node tissue is sampled and, based on the results, if cancer cell metastasis is found, lymph node dissection is performed wherein the lesion site is excised and the entire lymph node is removed including the sentinel lymph node and its surroundings. When metastasis of cancer cells is not found in the lymph nodes, only the sentinel lymph node tissue sampled for the diagnosis is excised, the surrounding lymph node being left without excision, in order to reduce the burden on the patient.

The proposed method for identifying the sentinel lymph node is to inject a magnetic fluid into the lesion site, allow a suitable period of time to elapse, and then detect the sentinel lymph node that has accumulated the injected magnetic fluid, using a magnetic sensor (see Patent Literatures 1 and 2, for example). A conventional magnetic fluid detecting device using such a method comprises a probe that detects magnetic fluid in the body when in contact with or adjacent to the body, and a controller connected to the probe via a cable. The detection results (for example, a magnetic flux density detection value) are outputted as a prescribed output (for example, a numerical display) from an output device provided in the controller.

Citation List

Patent Literature

    Patent Literature 1: Japanese Patent Publication No. 3847694
    Patent Literature 2: Japanese Patent Publication No. 3960558

Summary of Invention

Technical Problem

In the conventional magnetic fluid detecting device, however, the controller with the output device is set at a location separate from the probe, and therefore the detection results may be difficult to confirm, or the cable connecting the probe and the controller may interfere. Furthermore, devices that are brought into an operating room must be treated for sterilization, by procedures such as being entirely covered with a transparent sterile bag. However, since the conventional magnetic fluid detecting devices comprise a probe, controller and cable, their sterilization treatment has been time consuming.

Moreover, the magnetic fluid detecting device described in Patent Literature 1 has an actuator and an electromagnet that require high electric power input. Therefore, power consumption is increased, rendering the magnetic fluid detecting device unsuitable for use in environments with limited power supply. The magnetic fluid detecting device described in Patent Literature 2 is advantageous in terms of its lower power consumption because it does not require an actuator or electromagnet. However, since the controller is composed of an analog circuit, its circuit system is complex with numerous parts, and this has limited the extent to which its power consumption can be reduced.

Solution to Problem

The present invention has been devised with the object of solving the problems mentioned above, the invention according to claim 1 is a magnetic fluid detecting device that detects magnetic fluid injected into a living body and comprises a detector that detects magnetic fluid in the body when it is in contact with or adjacent to the body, an output device that outputs the detection results in a prescribed output form, a controller that controls the output device based on the detection value inputted from the detector, and a power supply module that supplies power to the detector, output device and controller using a battery as the power source, wherein the detector comprises a magnetic sensor and a permanent magnet disposed symmetrically around the magnetic sensor with the magnetic sensor as the center of symmetry, and generating flux toward the body, the permanent magnet having a magnetic flux density of approximately 0 when no magnetic fluid is present in its vicinity and forming a magnetic flux density blank region in which the magnetic flux density increases while approaching the magnetic fluid and the magnetic sensor being disposed in the magnetic flux density blank region, and the detector, the output device, the controller and the power supply module are integrally formed so as to allow holding with one hand.

The invention according to claim 2 is a magnetic fluid detecting device according to claim 1, wherein the controller is constructed using a microcontroller unit, and the magnetic sensor is connected to the controller via wiring that is not exposed to the exterior.

The invention according to claim 3 is a magnetic fluid detecting device according to claim 1 or 2, wherein the detector comprises a temperature sensor that detects the temperature of the magnetic sensor, and the controller comprises temperature compensating means that compensates the detected value of the magnetic sensor based on the detected value of the temperature sensor, and detection result output means that outputs the compensated detected value of the magnetic sensor in a prescribed output form from the output device.

The invention according to claim 4 is a magnetic fluid detecting device according to claim 3, wherein the controller comprises magnetic sensor reference value retaining means that retains the detected value of the magnetic sensor as a magnetic sensor reference value in response to a prescribed reference value resetting procedure, and temperature sensor reference value retaining means that retains the detected value of the temperature sensor as a temperature sensor reference value in response to the reference value resetting procedure, wherein the temperature compensating means compensates a magnetic sensor difference value, which is a difference between the current detected value of the magnetic sensor and the magnetic sensor reference value, based on a temperature sensor difference value, which is a difference between the current detected value of the temperature sensor and the temperature sensor reference value, and the detection result output means outputs the compensated magnetic sensor difference value in a prescribed form from the output device.

The invention according to claim 5 is a magnetic fluid detecting device according to any one of claims 1 to 4, wherein the output device is capable of outputting a sound, the controller comprises detection sound output control means that outputs the detected value of the magnetic sensor as a detection sound from the output device, and the detection sound output control means intermittently outputs a detection sound of a prescribed frequency at a prescribed cycle from the output device while varying the frequency and cycle of the detection sound in response to the detected value of the magnetic sensor.

The invention according to claim 6 is a magnetic fluid detecting device according to claim 5, wherein the detection sound output control means varies the frequency of the detection sound in an exponential curve in response to the detected value of the magnetic sensor.

Advantageous Effects of Invention

According to the invention of claim 1, since the detector, output device, controller and power supply module are integrated so as to allow holding with one hand, not only is it easy to confirm the detection results, but the magnetic fluid detection operation can also be carried out without interference from a cable. Furthermore, since the integrated magnetic fluid detecting device can be easily covered with a sterile bag, sterilizing treatment is facilitated when it is to be brought into an operating room. Moreover, since magnetic fluid is detected by a combination of a permanent magnet and a magnetic sensor, the level of power consumption is greatly reduced compared to using an actuator or electromagnet, and as a result it is possible to drive the device with a battery, and also to extend the usable time period with the battery. In addition, since the magnetic sensor is disposed in a magnetic flux density blank region, it is possible for variations in magnetic flux density during approach toward magnetic fluids to be detected with high sensitivity, despite the low level of power consumption.

Furthermore, according to the invention of claim 2, since the controller is constructed using a microcontroller unit (digital circuit), it is possible to not only drastically reduce the number of parts compared to a controller constructed with an analog circuit, but also to reduce power consumption to a level allowing driving with a battery. It is an issue when performing digitalization that, with conversion of an analog signal obtained from a detector to a digital signal, the precision of the signal is lowered by error (quantization error), such that detection becomes difficult with low magnetic fluid quantities. However, according to the invention, the detector and controller are integrated and connected to the exterior with non-exposed wiring, thereby allowing reduction in noise introduced by wiring, and allowing compensation for reduced signal precision caused by quantization error.

Furthermore, according to the invention of claim 3, since the detected value of the magnetic sensor is compensated based on the detected value of the temperature sensor, it is possible to suppress variation in the detected value (output value) by temperature change despite the temperature dependence of the magnetic sensor, and thus to detect magnetic fluid with high precision.

Moreover, according to the invention of claim 4, since the degree of variation in the detected value is outputted based on a reference value that is the detected value of the magnetic sensor when reference value resetting means has been operated (the magnetic sensor difference value), it is possible to reduce the effects of individual differences between magnetic sensors and errors due to the external environment such as geomagnetism and noise, and to increase the detection accuracy for magnetic fluid. In addition, since the magnetic sensor difference value is compensated based on the degree of variation in the detected value based on a reference value that is the detected value of the temperature sensor when the reference value resetting means has been operated (the temperature sensor difference value), it is possible to precisely compensate the magnetic sensor difference value without being affected by individual differences between temperature sensors.

Furthermore, according to the invention of claim 5, since the detected value of the magnetic sensor is outputted as a detection sound and the frequency and cycle of the detection sound are varied in response to the detected value of the magnetic sensor, it is possible to easily recognize the detected value of the magnetic sensor based on the frequency and cycle of the detection sound.

Also, according to the invention of claim 6, since the frequency of the detection sound changes as an exponential curve in response to the detected value of the magnetic sensor, it is possible to be reliably informed of the approach of magnetic fluid when the frequency of the detection sound changes significantly as the detected value of the magnetic sensor increases while approaching magnetic fluid.

Brief Description of Drawings

[Fig. 1] Fig. 1 is a block diagram showing the construction of a magnetic fluid detecting device according to an embodiment of the invention.
[Fig. 2] Fig. 2 is a general side view showing the construction of a magnetic fluid detecting device according to an embodiment of the invention.
[Fig. 3] Fig. 3 is a magnified cross-sectional view showing the detector of a magnetic fluid detecting device according to an embodiment of the invention, where (A) is a cross-sectional view along X-X of Fig. 2, and (B)* is a cross-sectional view along Y-Y of Fig. 2.
[Fig. 4] Fig. 4 is an illustration showing the principle of detection of a magnetic fluid detecting device according to an embodiment of the invention, where (A) is a schematic diagram showing magnetic flux line distribution in the absence of magnetic fluid, and (B) is a schematic diagram showing magnetic flux line distribution during approach of magnetic fluid.
[Fig. 5] Fig. 5 is a flowchart showing the detection control procedure with a controller according to an embodiment of the invention.
[Fig. 6] Fig. 6 is an illustration showing the relationship between detected magnetic flux density and detection sound in a magnetic fluid detecting device according to an embodiment of the invention, where (A) is a graph showing the relationship between detected magnetic flux density and detection sound frequency, and (B) is a graph showing the relationship between detected magnetic flux density and detection sound pulse cycle.

Description of Embodiments

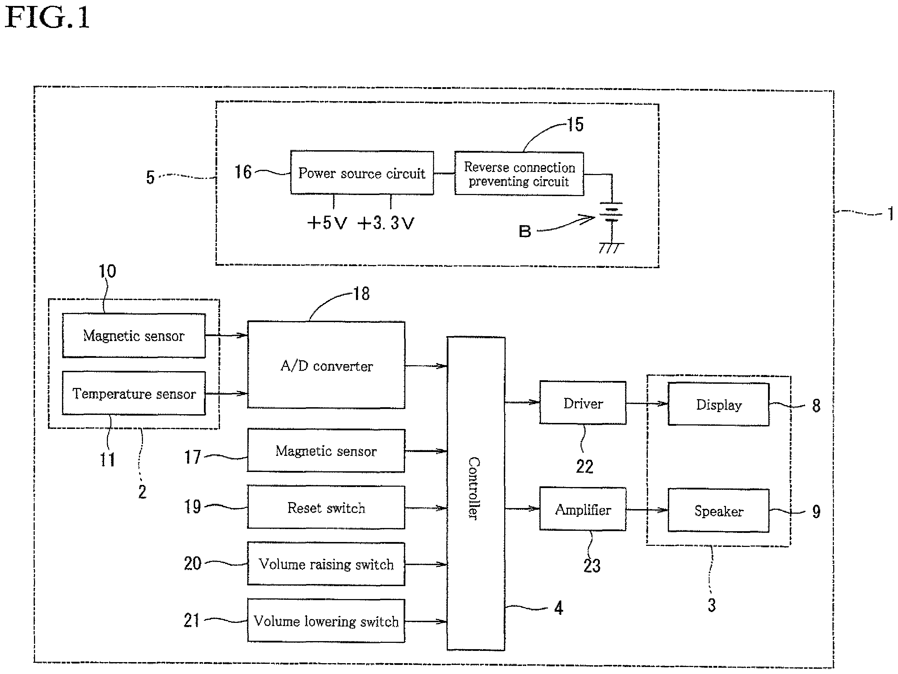

An embodiment of the invention will now be described with reference to the accompanying drawings. In Fig. 1, 1 is a magnetic fluid detecting device that detects magnetic fluid that has been injected into a living body, the magnetic fluid detecting device 1 comprising a detector 2 that detects magnetic fluid in the body when it is in contact with or adjacent to the body, an output device 3 that outputs the detection results in a prescribed output form, a controller 4 that controls the output device 3 based on the detected value inputted from the detector 2, and a power supply module 5 that supplies power to the detector 2, output device 3 and controller 4, using a battery B as the power source.

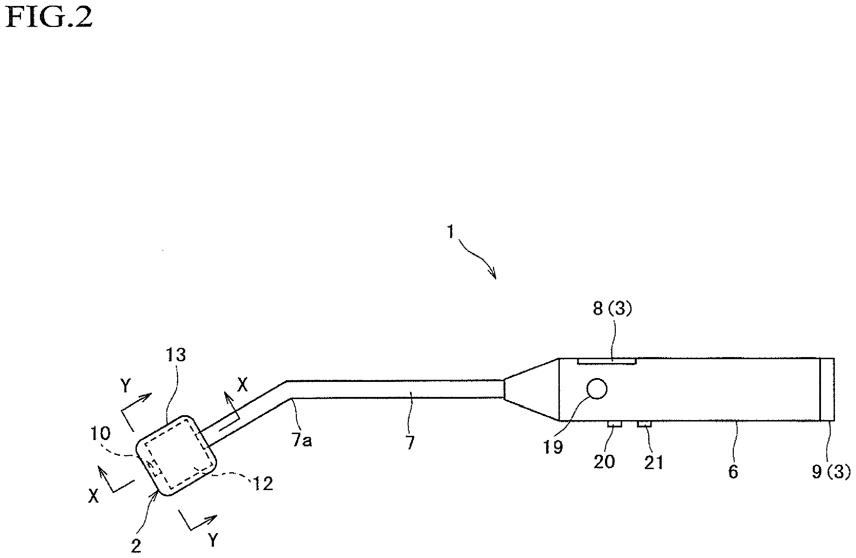

As shown in Fig. 1 and Fig. 2, the detector 2, output device 3, controller 4 and power supply module 5 composing the magnetic fluid detecting device 1 are integrated so as to allow holding with one hand. More specifically, the magnetic fluid detecting device 1 comprises a case 6 in a grippable (cylindrical) form that can be held with one hand, the controller 4 and power supply module 5 being housed inside the case 6. At the front end of the case 6 there is provided a non-magnetic shaft 7, with the detector 2 being provided at the tip section of the non-magnetic shaft 7. For this embodiment, a display 8 of the output device 3 is provided on the periphery of the case 6, and a speaker 9 of the output device 3 is provided at the rear end of the case 6, but such arrangements are optional, as long as they are integrated with the magnetic fluid detecting device 1. The non-magnetic shaft 7 is a hollow shaft having a hollow section through which wiring C to connect the detector 2 and controller 4 is inserted, and a curved section 7a is formed at the leading edge for appropriate orientation of the detector 2.

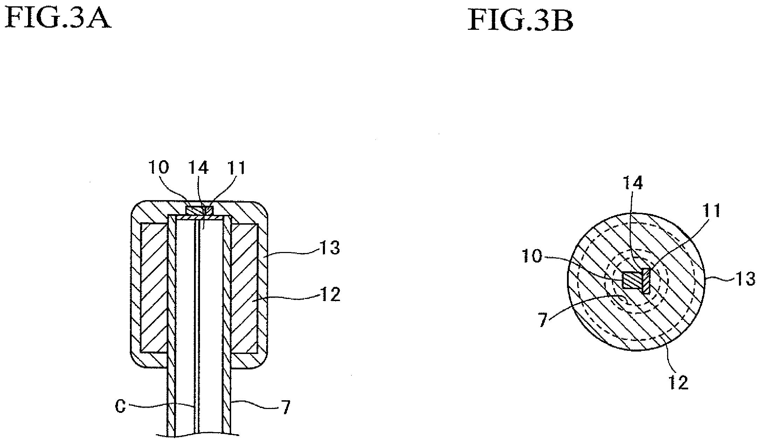

As shown in Fig. 3, the detector 2 comprises a magnetic sensor 10, a temperature sensor 11 and a permanent magnet 12, at the tip section of the non-magnetic shaft 7, and an insulating member 13 covering the magnetic sensor 10, temperature sensor 11 and permanent magnet 12. The magnetic sensor 10 of this embodiment is a Hall element and the temperature sensor 11 is a thermistor, but other detecting elements may be used instead.

The permanent magnet 12 is situated in a symmetrical manner around the magnetic sensor 10 with the magnetic sensor 10 as the center of symmetry, and it generates magnetic flux toward the body. Specifically, a cylindrical rare earth magnet is used as the permanent magnet 12, being placed with the N-pole end at the front end and the S-pole end at the rear end. A rare earth magnet is a super-ferromagnetic permanent magnet with high magnetic properties (residual magnetic flux density (Br), coercive force (bHc, iHc), maximum energy product (BHmax), etc.), and for example, general purpose magnets produced using the rare earth element samarium (Sm) or neodymium (Nd) may be used.

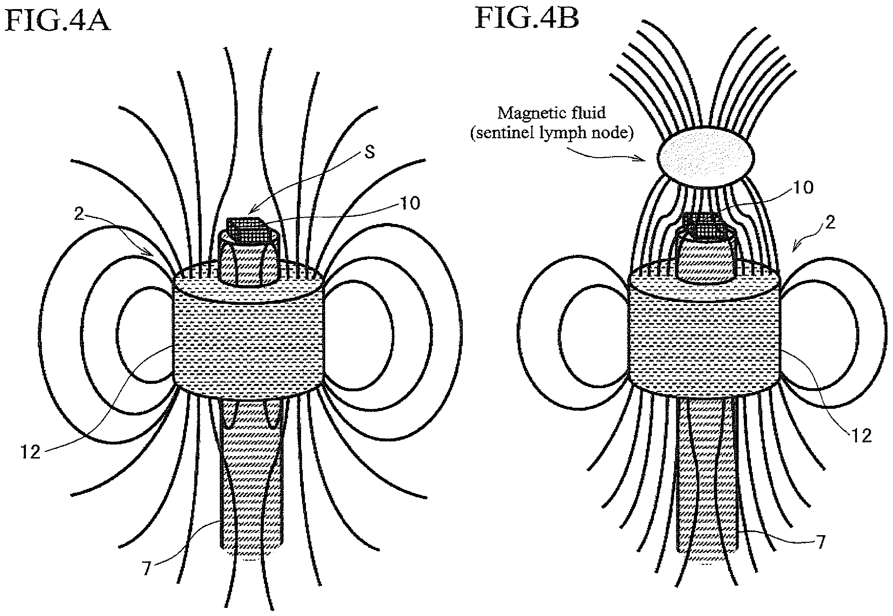

When the permanent magnet 12 is disposed as described above, a magnetic flux density blank region S is formed on the central axis of the permanent magnet 12. As shown in Fig. 4, when a magnetic fluid is not in the vicinity, the magnetic flux density blank region S does not produce magnetic flux lines and has a magnetic flux density of approximately 0, but magnetic flux lines aggregate as the magnetic fluid is approached, creating a region of increasing magnetic flux density. Placing the magnetic sensor 10 in this region of high magnetic flux density variation results in increased detection sensitivity of the magnetic fluid by the magnetic sensor 10.

For this embodiment, incidentally, as shown in Fig. 4, the magnetic flux density blank region S is defined as the region formed at the boundary section between the magnetic flux lines oriented from the N-pole (front end side) of the cylindrical permanent magnet 12 toward the S-pole (rear end side) of the permanent magnet 12 through the inner perimeter side of the permanent magnet 12, and the magnetic flux lines oriented from the N-pole of the permanent magnet 12 toward the S-pole of the permanent magnet 12 through the outer perimeter side of the permanent magnet 12.

The temperature sensor 11 is disposed adjacent to the magnetic sensor 10 and detects the temperature of the magnetic sensor 10. During this time, a heat conducting member 14 with high thermal conductivity (for example, a metal sheet or a heat-conductive adhesive) is preferably situated between the magnetic sensor 10 and the temperature sensor 11. The reason for this is to allow temperature changes at the magnetic sensor 10 to be rapidly transmitted to the temperature sensor 11 through the heat conducting member 14, thereby allowing precise detection of the temperature at the magnetic sensor 10 in real time.

The insulating member 13 is formed, for example, of a resin material with low thermal conductivity, and it covers the magnetic sensor 10, temperature sensor 11 and permanent magnet 12. Also, the insulating member 13 controls temperature change at the magnetic sensor 10 due to body heat, being situated between the body and the magnetic sensor 10 and temperature sensor 11 when magnetic fluid that has been injected into a living body is to be detected. The insulating member 13 also functions as a protective member for the magnetic sensor 10 and temperature sensor 11. For example, by having the insulating member 13 disposed as a protective member, it prevents damage to the magnetic sensor 10 and temperature sensor 11 when the permanent magnet 12 composed of a rare earth magnet has been adsorbed onto a magnetic body by its powerful magnetic force.

As shown in Fig. 1, the power supply module 5 comprises a battery B as the power source, a reverse connection preventing circuit 15 that prevents reverse connection with the battery B, and a power source circuit 16 that transforms the voltage of the battery B to the required voltage for the detector 2, output device 3 and controller 4. The battery B may be a dry cell that is replaced as the remaining battery power decreases, or it may be a charge battery that is charged as the remaining battery power decreases.

As shown in Fig. 1, the controller 4 is a control unit constructed using a microcontroller unit (for example, a single chip microcomputer), having connected at the input end, in addition to the aforementioned magnetic sensor 10 and temperature sensor 11, also a magnetic sensor 17 connected via an A/D converter 18 to compensate for geomagnetism, and switches including a reset switch 19 also serving as a power switch, and a volume raising switch 20 and a volume lowering switch 21 for raising and lowering of the volume of the speaker 9. At the output end of the controller 4, the display 8 (for example, a 7-segment LED display or a liquid crystal display) for output of the detected value of the magnetic sensor 10 in numerical form is connected via a driver 22, and the speaker 9 for output of the detected value of the magnetic sensor 10 as a detection sound is connected via an amplifier 23.

The magnetic sensor 17 for compensation of geomagnetism is situated in a case 6 that is to detect geomagnetism, the detection error caused by geomagnetism being eliminated by taking the difference from the detected value of the magnetic sensor 10, but compensation for geomagnetism will not be explained in detail here. Also, power source ON/OFF control by the action of holding down the reset switch 19, and volume control by operation of the volume raising switch 20 and volume lowering switch 21, will not be explained in detail here.

The controller 4 cooperates with a program written in ROM, to function as temperature compensating means whereby the detected value of the magnetic sensor 10 is compensated based on the detected value of the temperature sensor 11, detection result output means by which the compensated detected value of the magnetic sensor 10 is outputted in a desired form, and detection sound output control means whereby the detected value of the magnetic sensor 10 is outputted from the output device 3 (speaker 9) as a detection sound.

The formula used for compensation of the detected value of the magnetic sensor 10 based on the detected value of the temperature sensor 11 may be the following. Here, V' is the detected value of the magnetic sensor 10 after compensation (output value), V is the detected value of the magnetic sensor 10 before compensation, T is the detected value of the temperature sensor 11, B is the magnetic field (detected value of the magnetic sensor 10), and $\alpha$, $\beta$, $\gamma$ and 8 are compensation coefficients.

$$\text{Compensation formula 1: } V' = V - \alpha T$$

$$\text{Compensation formula 2: } V' = V - \alpha T + \beta T^2$$

$$\text{Compensation formula 3: } V' = (1 - \gamma T)\, V$$

$$\text{Compensation formula 4: } V' = V - \alpha T - \delta BT$$

The controller 4 of this embodiment sets reference values for both sensors 10, 11 and performs compensation using the difference between the reference value and the current detected value, in order to reduce the effect of individual differences for the magnetic sensor 10 and temperature sensor 11 and sources of error such as noise. More specifically, the controller 4 of this embodiment functions as magnetic sensor reference value retaining means that retains a detected value V of the magnetic sensor 10 in response to operation of the reset switch 19, as the magnetic sensor reference value $V_0$, and as temperature sensor reference value retaining means that retains a detected value T of the temperature sensor 11 in response to operation of the reset switch 19, as the temperature sensor reference value $T_0$. The temperature compensating means compensates a magnetic sensor difference value, which is a difference between the current detected value V of the magnetic sensor 10 and a magnetic sensor reference value $V_0$, based on the temperature sensor difference value which is a difference between the current detected value T of the temperature sensor 11 and the temperature sensor reference value $T_0$. The detection result output means outputs the compensated magnetic sensor difference value $\Delta V'$ in the desired form.

The formula used for this compensation may be the following.

$$\text{Compensation formula 5: } \Delta V' = (V - V_0) - \alpha(T - T_0)$$

$$\text{Compensation formula 6: } \Delta V' = (V - V_0) - \alpha(T - T_0) + \beta(T - T_0)^2$$

$$\text{Compensation formula 7: } \Delta V' = \{1 - \gamma\,(T - T_0)\}(V - V_0)$$

$$\text{Compensation formula 8: } \Delta V' = (V - V_0) - \alpha(T - T_0) - \delta B(T - T_0)$$

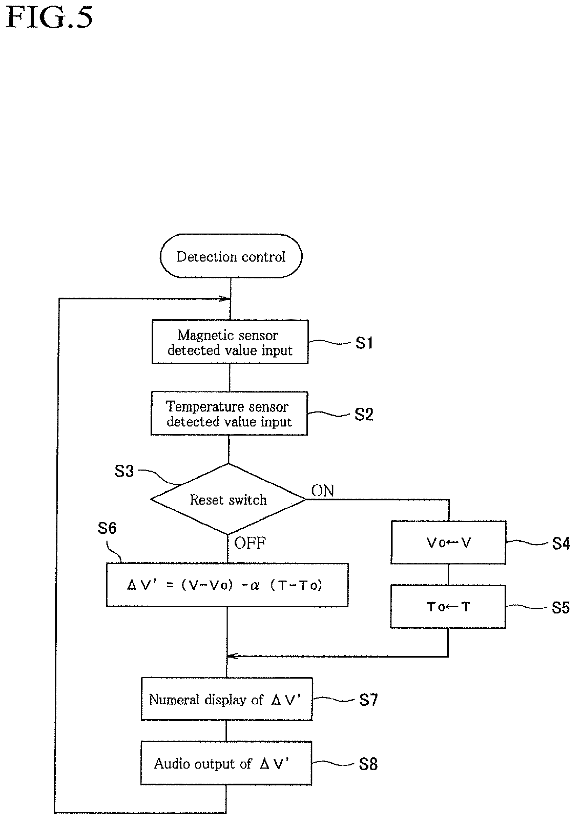

The control procedure when compensation is performed using the formula 5 will now be explained with reference to Fig. 5.

As shown in Fig. 5, for detection control, first the detected value V of the magnetic sensor 10 and the detected value T of the temperature sensor 11 are inputted (S1, S2), and operation of the reset switch 19 is judged (S3). When the judgment result is "ON," the detected value V of the magnetic sensor 10 is set to the magnetic sensor reference value $V_0$ (S4: magnetic sensor reference value retaining means), while the detected value T of the temperature sensor 11 is set to the temperature sensor reference value $T_0$ (S5: temperature sensor reference value retaining means).

When setting of the reference values has been completed, or if the reset switch 19 is judged to be "OFF," then the magnetic sensor difference value, which is the difference between the current detected value V of the magnetic sensor 10 and the magnetic sensor reference value $V_0$, is compensated based on the temperature sensor difference value, which is the difference between the current detected value T of the temperature sensor 11 and the temperature

sensor reference value $T_0$ (S6: temperature compensating means).

Next, the compensated magnetic sensor difference value $\Delta V'$ is displayed as a numerical value on the display 8 (S7: detection result output means) and outputted as a detection sound from a speaker 9 (S8: detection result output means, detection sound output control means). These processing steps (S1 to S8) are then repeated in an endless loop until a power source OFF operation is performed. For this example, the magnetic sensor difference value $\Delta V'$ is converted to magnetic flux density (units: $\mu$T) and the magnetic flux density is displayed on the display 8 as a numerical value, but the display may instead be as a bar graph or the like.

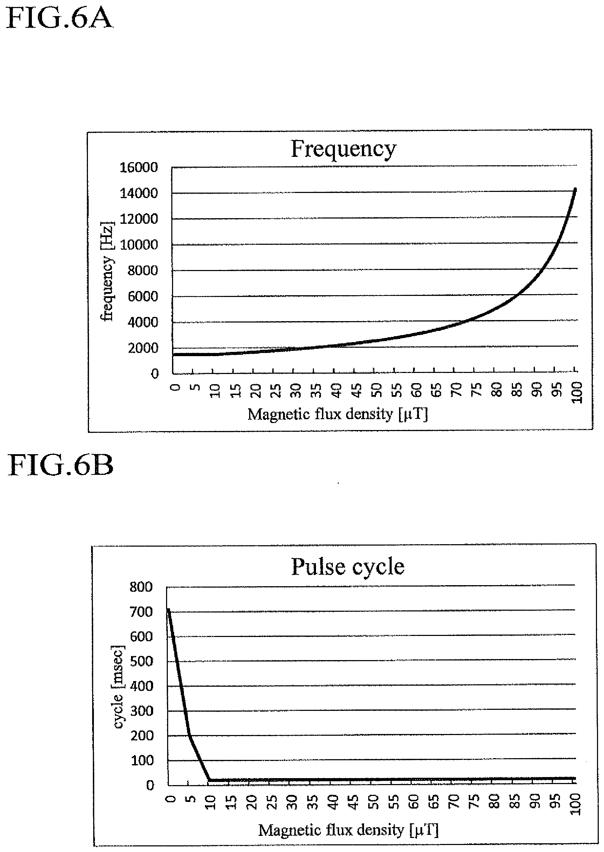

As shown in Fig. 6, the detection sound output control means intermittently outputs a detection sound of the prescribed frequency from the speaker 9 at a prescribed cycle, while varying the frequency and cycle of the detection sound in response to the detected value of the magnetic sensor 10 (detected magnetic flux density value).

More specifically, in the detection sound output control means of this embodiment, as shown in Fig. 6(B), the output cycle (pulse cycle) of the detection sound is inversely proportional to the magnetic flux density in the region of very low magnetic flux density ($\leq$10 $\mu$T), and is constant regardless of the magnetic flux density in the region exceeding this region. In other words, in the initial state with no detection of magnetic fluid, a detection sound with a large output interval is outputted ("beep ... beep ... beep"), but when detection of magnetic fluid begins, a detection sound with a small output interval is outputted ("beepbeepbeep"), thereby signaling that detection of magnetic fluid has begun. The formula for determining the output cycle for the detection sound from the magnetic flux density is shown below. The output cycle (units: msec) is represented as y, and the magnetic flux density (units: $\mu$T) as MFD.

$$\text{For } [0 \leq MFD \leq 5]: y = -103 * MFD + 710$$

$$\text{For } [5 \leq MFD \leq 10]: y = -35 * MFD + 380$$

$$\text{For } [10 \leq MFD]: y = 20$$

The detection sound output control means of this embodiment varies the frequency of the detection sound in an exponential curve in response to the magnetic flux density, as shown in Fig. 6(A). That is, when the magnetic flux density increases while approaching a magnetic fluid, the frequency of the detection sound is greatly varied to reliably signal the approach of the magnetic fluid. The formula for determining the frequency of the detection sound from the magnetic flux density is shown below. The frequency (units: Hz) is represented as f.

$$[\text{For } MFD < 10]: f = 1501$$

$$\text{For } [10 \leq MFD \leq 100]: f = 1365.56/(1 - 0.0090366 * MFD)$$

$$\text{For } [100 < MFD]: f = 14178$$

In the magnetic fluid detecting device 1 of this embodiment having the aforementioned construction, the detector 2, output device 3, controller 4 and power supply module 5 are integrated so as to allow holding with one hand, and therefore not only is it easy to confirm the detection results, but the magnetic fluid detection operation can also be carried out without interference from a cable. Furthermore, since the integrated magnetic fluid detecting device 1 can be easily covered with a sterile bag, sterilizing treatment is facilitated when it is to be brought into an operating room.

Moreover, even though a battery B is used as the power source, since the magnetic fluid is detected in combination with a permanent magnet 12 and magnetic sensor 10, the power consumption can be suppressed compared to a combination of an electromagnet and a magnetic sensor, and the usable time with the battery B can be extended.

In addition, since the magnetic sensor 10 is disposed in the magnetic flux density blank region S, it is possible for variations in magnetic flux density during approach toward magnetic fluids to be detected with high sensitivity, despite the reduced electric power.

Furthermore, since the controller 4 is constructed using a microcontroller unit (digital circuit), it is possible to not only drastically reduce the number of parts compared to a controller 4 constructed with an analog circuit, but also to reduce power consumption to a level allowing driving with a battery.

It is an issue when performing digitalization that, with conversion of an analog signal obtained from a detector 2 to a digital signal, the precision of the signal is lowered by error (quantization error), such that detection becomes difficult with low magnetic fluid quantities. However, according to the invention, the detector 2 and controller 4 are integrated and connected to the exterior with non-exposed wiring C, thereby allowing reduction in noise that is introduced by the wiring C, and allowing compensation for reduced signal precision caused by quantization error.

Furthermore, since the detected value of the magnetic sensor 10 is compensated based on the detected value of the temperature sensor 11, it is possible to suppress variation in the detected value by temperature change despite the temperature dependence of the magnetic sensor 10, and thus to detect magnetic fluid with high precision.

Moreover, since the degree of variation in the detected value is outputted based on a reference value that is the detected value of the magnetic sensor 10 when resetting has been performed (the magnetic sensor difference value), it is possible to reduce the effects of individual differences between magnetic sensors 10 and errors due to noise and the like, and to increase the detection accuracy for magnetic fluid.

In addition, since the magnetic sensor difference value is compensated based on the degree of variation in the detected value with respect to a reference value that is the detected value of the temperature sensor 11 when resetting has been performed (the temperature sensor difference value), it is possible to precisely compensate the magnetic sensor difference value without being affected by individual differences between temperature sensors 11.

Furthermore, since the detected value of the magnetic sensor 10 is outputted as a detection sound and the frequency and cycle of the detection sound are varied in response to the detected value of the magnetic sensor 10, it is possible to easily recognize the detected value of the magnetic sensor 10 based on the frequency and cycle of the detection sound.

Also, since the frequency of the detection sound changes as an exponential curve in response to the detected value of the magnetic sensor 10, it is possible to be reliably informed of the approach of magnetic fluid when the frequency of the detection sound changes significantly as the detected value of the magnetic sensor 10 has increased while approaching magnetic fluid.

Description of Symbols

1 Magnetic fluid detecting device
2 Detector
3 Output device
4 Controller
5 Power supply module
8 Display
9 Speaker
10 Magnetic sensor
11 Temperature sensor
12 Permanent magnet
19 Reset switch
B Battery
S Magnetic flux density blank region
Fig. 1

4    Controller
8    Display screen
9    Speaker
10   Magnetic sensor
11   Temperature sensor
15   Reverse connection preventing circuit
16   Power source circuit
17   Magnetic sensor
18   A/D converter

19   Reset switch
20   Volume raising switch
21   Volume lowering switch
22   Driver

23     Amplifier

Fig. 4
Magnetic fluid (sentinel lymph node)
Fig. 5
Detection control

S1     Magnetic sensor detected value input
S2     Temperature sensor detected value input
S3     Reset switch
ON
OFF
S7     Numeral display of ΔV'
S8     Audio output of ΔV'

Fig. 6

(A)
Frequency
Frequency [Hz]
Magnetic flux density [$\mu$T]
(B)
Pulse cycle
Cycle [msec]
Magnetic flux density [$\mu$T]

**Claims**

1. A magnetic fluid detecting device that detects magnetic fluid injected into a living body, comprising:

   a detector that detects magnetic fluid in the body when it is in contact with or adjacent to the body,
   an output device that outputs the detection results in a prescribed output form,
   a controller that controls the output device based on the detection value inputted from the detector, and
   a power supply module that supplies power to the detector, output device and controller using a battery as the power source,
   wherein the detector comprises:

   a magnetic sensor and
   a permanent magnet disposed symmetrically around the magnetic sensor with the magnetic sensor as the center of symmetry, and generating flux toward the body,
   the permanent magnet having a magnetic flux density of approximately 0 when no magnetic fluid is present in its vicinity and forming a magnetic flux density blank region in which the magnetic flux density increases while approaching the magnetic fluid and
   the magnetic sensor being disposed in the magnetic flux density blank region, and
   the detector, the output device, the controller and the power supply module are integrally formed so as to allow holding with one hand.

2. A magnetic fluid detecting device according to claim 1, wherein
   the controller is constructed using a microcontroller unit, and
   the magnetic sensor is connected to the controller via wiring that is not exposed to the exterior.

3. A magnetic fluid detecting device according to claim 1 or 2, wherein:

   the detector comprises a temperature sensor that detects the temperature of the magnetic sensor, and
   the controller comprises:

   temperature compensating means that compensates the detected value of the magnetic sensor based on the detected value of the temperature sensor, and
detection result output means that outputs the compensated detected value of the magnetic sensor in a prescribed output form from the output device.

4. A magnetic fluid detecting device according to claim 3, wherein:

the controller comprises
magnetic sensor reference value retaining means that retains the detected value of the magnetic sensor as a magnetic sensor reference value in response to a prescribed reference value resetting procedure, and
temperature sensor reference value retaining means that retains the detected value of the temperature sensor as a temperature sensor reference value in response to the reference value resetting procedure, wherein
the temperature compensating means compensates a magnetic sensor difference value, which is a difference between the current detected value of the magnetic sensor and the magnetic sensor reference value, based on a temperature sensor difference value, which is a difference between the current detected value of the temperature sensor and the temperature sensor reference value, and
the detection result output means outputs the compensated magnetic sensor difference value in a prescribed form from the output device.

5. A magnetic fluid detecting device according to any one of claims 1 to 4, wherein:

the output device is capable of outputting a sound,
the controller comprises detection sound output control means that outputs the detected value of the magnetic sensor as a detection sound from the output device, and
the detection sound output control means intermittently outputs a detection sound of a prescribed frequency at a prescribed cycle from the output device while varying the frequency and cycle of the detection sound in response to the detected value of the magnetic sensor.

6. A magnetic fluid detecting device according to claim 5, wherein the detection sound output control means varies the frequency of the detection sound in an exponential curve in response to the detected value of the magnetic sensor.

# FIG.1

# FIG.2

FIG.3A

FIG.3B

FIG.4A

FIG.4B

Magnetic fluid
(sentinel lymph node)

# FIG.5

```
           ┌─────────────────────┐
           │  Detection control  │
           └──────────┬──────────┘
                      │
    ┌─────────────────┤
    │                 ↓
    │         ┌──────────────────┐
    │         │ Magnetic sensor  │──── S1
    │         │detected value input│
    │         └────────┬─────────┘
    │                  │
    │         ┌──────────────────┐
    │         │Temperature sensor│──── S2
    │         │detected value input│
    │         └────────┬─────────┘
    │                  │
  S3 ─────             ◇
    │         ╱                 ╲       ON
    │        ◇   Reset switch     ◇──────────────┐
    │         ╲                 ╱                 │
  S6           ◇                                  ↓
    │          │ OFF                   ┌──────────────────┐
    │          ↓                       │    Vo←V          │──── S4
    │  ┌──────────────────────┐        └────────┬─────────┘
    │  │ ΔV' = (V−Vo) −α (T−To)│               │
    │  └──────────┬───────────┘        ┌──────────────────┐
    │             │                    │    To←T          │──── S5
    │             │←───────────────────└──────────────────┘
    │             ↓
    │  ┌──────────────────────┐
    │  │ Numeral display of ΔV'│──── S7
    │  └──────────┬───────────┘
    │             │
    │  ┌──────────────────────┐
    │  │ Audio output of ΔV'   │──── S8
    │  └──────────┬───────────┘
    │             │
    └─────────────┘
```

$$\Delta V' = (V - Vo) - \alpha\ (T - To)$$

15

## FIG.6A

## FIG.6B

## INTERNATIONAL SEARCH REPORT

| | International application No. |
|---|---|
| | PCT/JP2015/081812 |

### A. CLASSIFICATION OF SUBJECT MATTER
*A61B5/05*(2006.01)i

According to International Patent Classification (IPC) or to both national classification and IPC

### B. FIELDS SEARCHED

Minimum documentation searched (classification system followed by classification symbols)
A61B5/00

Documentation searched other than minimum documentation to the extent that such documents are included in the fields searched
| | | | |
|---|---|---|---|
| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2008-206578 A (Kabushiki Kaisha Kyoshin), 11 September 2008 (11.09.2008), paragraphs [0006] to [0019] (Family: none) | 1-6 |
| Y | WO 2011/067576 A1 (ENDOMAGNETICS LTD.), 09 June 2011 (09.06.2011), page 22, line 20 to page 23, line 7 & JP 2013-512713 A paragraph [0062] & US 2011/0133730 A1 | 1-6 |
| Y | JP 2014-163825 A (Asahi Kasei EMD Corp.), 08 September 2014 (08.09.2014), paragraphs [0015] to [0034] (Family: none) | 3-4 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 09 February 2016 (09.02.16) | 23 February 2016 (23.02.16) |

| Name and mailing address of the ISA/ Japan Patent Office 3-4-3,Kasumigaseki,Chiyoda-ku, Tokyo 100-8915,Japan | Authorized officer |
|---|---|
| | Telephone No. |

Form PCT/ISA/210 (second sheet) (January 2015)

**INTERNATIONAL SEARCH REPORT**

International application No.

PCT/JP2015/081812

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2013-120538 A  (Nidec Sankyo Corp.), 17 June 2013 (17.06.2013), paragraphs [0017] to [0084] (Family: none) | 3-4 |
| Y | JP 2012-042259 A  (Kohden Co., Ltd.), 01 March 2012 (01.03.2012), paragraphs [0021] to [0098] (Family: none) | 3-4 |
| A | JP 2014-188122 A  (Akita Prefectural Hospital Organization), 06 October 2014 (06.10.2014), paragraphs [0018] to [0113] (Family: none) | 1-6 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3847694 B

- JP 3960558 B